(19) Europäisches Patentamt
European Patent Office
Office européen des brevets

(11) Publication number: 0 127 076 A2

(12) EUROPEAN PATENT APPLICATION

(21) Application number: 84105601.3

(22) Date of filing: 17.05.84

(51) Int. Cl.[3]: C 07 D 233/64
C 12 P 17/10
//(C12P17/10, C12R1/465)

(30) Priority: 23.05.83 JP 89075/83

(43) Date of publication of application: 05.12.84 Bulletin 84/49

(84) Designated Contracting States: AT BE CH DE FR GB IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
4-23, Toyotama-Kita Nerima-ku
Tokyo(JP)

(72) Inventor: Takeuchi, Tomio
5-1-11, Higashi-gotanda
Shinagawa-ku Tokyo(JP)

(72) Inventor: Aoyagi, Takaaki
3-3-6, Honkugenuma
Fujisawa-city Kanagawa Prefecture(JP)

(72) Inventor: Hamada, Masa
1-26, Naito-cho
Shinjuku-ku Tokyo(JP)

(72) Inventor: Ogawa, Keiji
715-204, Uchikoshi-cho
Hachioji-city Tokyo(JP)

(72) Inventor: Iinuma, Hironobu
3-35-6-301, Shirako
Wako-city Saitama Prefecture(JP)

(72) Inventor: Ohbayashi, Akira
1-1-122, Nanryo-cho
Uji-city Kyoto Prefecture(JP)

(72) Inventor: Hachisu, Mitsugu
1-5-15, Minami
Meguro-ku Tokyo(JP)

(72) Inventor: Kawamura, Kenji
2-4-5, Ishigamidai Ohiso-machi
Naka-gun Kanagawa Prefecture(JP)

(72) Inventor: Niida, Taro
127, Nakakibohgaoka Asahi-ku
Yokohama-city Kanagawa Prefecture(JP)

(74) Representative: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

# (54) Histargin, a microbiological process for its preparation, its use as a medicament, and the strain Streptomyces roseoviridis having the ability to produce histargin.

(57) The present invention relates to a novel physiologically active substance, histargin, a microbiological process for the preparation thereof, a novel strain having the ability to produce histargin, and the use of histargin as a medicament having analgesic and immuno-potentiating activity.

EP 0 127 076 A2

./...

Croydon Printing Company Ltd.

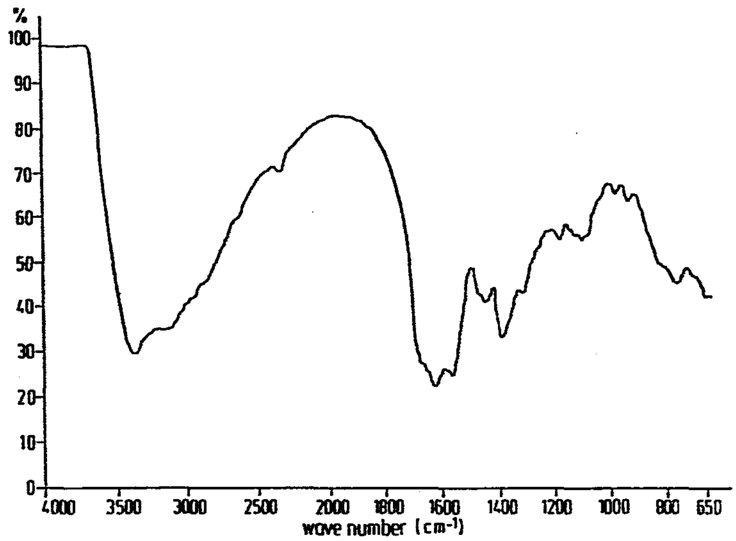
%
100
90
80
70
60
50
40
30
20
10
0
4000
3500
3000
2500
2000
1800
1600
1400
1200
1000
800
650
wave number (cm-1)

The present invention, relates to a novel physiologically active substance, histargin, of the following formula

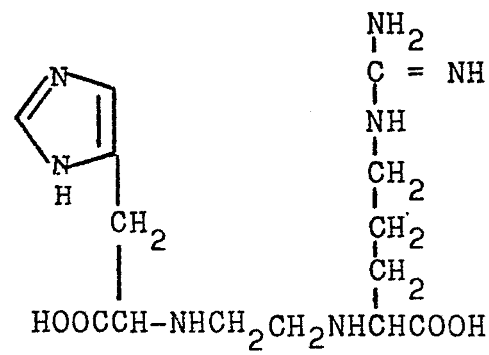

or a salt or ester or hydrate thereof.

Histargin can be collected as a white powder by filtrating the culture broth of a histargin-producing microorganism, and fractionating the filtrate by chromatography or the like using an ion-exchange resin such as Amberlite IRC 50, as shown in the Examples to appear later.

Histargin monohydrate has the following properties:

Melting point: 161-165°C

Molecular weight by mass spectrometry: 355

Elemental analysis: C: 45.53 %, H: 7.53 %, N: 26.28 %, O: 20.80 %, (these values agree with the molecular formula $C_{14}H_{25}N_7O_4 \cdot H_2O$)

Specific rotation: $[\alpha]^{20}_{D} = 36.3°$ (c=1, in constant boiling point hydrochloric acid)

Infrared absorption spectrum (KBr):

Given in the attached drawing. Shows characteristic absorption bands at 3375, 1670, 1630, 1580, 1450, 1400, 1320, 1180, 1100, 980, 940 and 760 ($cm^{-1}$).

Proton NMR spectrum (in heavy water solution, 100 MHZ): Shows absorption at 1.8-2.3 ($CH_2$ x 2), 3.2-3.5

$(CH_2 \times 3)$, 3.5 - 3.65 $(CH_2)$, 3.65-3.83 (CH), 3.85-4.05 (CH), 7.3 - 7.4 (-CH=), and 8.05-8.15 (-CH=) ppm.

Histargin shows enzyme-inhibiting activity against carboxypeptidase B and enkephalinase B. It exhibits further the activity to potentiate cell-mediated immunity and analgesic and analgesia-potentiating activities.

The present invention relates further to a process for producing histargin, which comprises cultivation a histargin-producing strain of the genus Streptomyces to produce and accumulate histargin in the culture broth, and then recovering histargin.

An example of the strain for producing histargin according to the process of this invention is Streptomyces roseovirides MF118-A5 which was isolated from soil collected in Itami City, Hyogo Prefecture, Japan. This strain was deposited on April 18, 1983 with the Fermentation Research Institute, Agency of Industrial Science and Technolgoy, Ministry of International Trade and Industry of Japan, where it was kept as FERM-P No.7043 (Accession No. FERM BP-527).

The microbiological characteristics of this strain (MF118-A5) are described below.

1. Morphology

Microscopically, substrate mycelia of MF118-A5 are branched and extend straight aerial hyphae. No whirls are observed on the aerial hyphae. Chains of mature spores include those having more than 10 spores. The spores range in size from about 0.4 to 0.6 x 1.6 to 1.8 microns, and their surfaces are smooth.

2. Culture characteristics on various media

In the following disclosure, the standards indicated in square brackets [ ] to describe colors comply with the Color Harmony Manual issued by the Container Corporation of America.

(1) On sucrose-nitrate agar medium (cultured at 27°C): Growth is colorless, and white aerial mycelia

slightly develop on the growth. No soluble pigment is produced.

(2) On glucose-asparagine agar medium (cultured at 27°C): Growth is pale yellow, and pale pink [5 ca, Flesh pink] aerial mycelia develop on the growth. No soluble pigment is produced.

(3) On glycerol-asparagine agar medium (ISP-5, cultured at 27°C): Growth is pale brown [3 gc, Lt Tan], and white to pale pink [6 ca, Flesh pink] aerial mycelia develop on the growth. Slightly brownish soluble pigment is produced.

(4) On starch-inorganic salts agar medium (ISP-4, cultured at 27°C): Growth is pale yellowish brown, and pale brownish [4 ec, Bisgue) aerial mycelia develop on the growth. Slightly brownish soluble pigment is produced.

(5) On tyrosine agar medium (ISP-7, cultured a 27°C): Growth is pale yellowish brown, and brownish white (3 ba, pearl) aerial mycelia develop on the growth. Slightly brownish soluble pigment is produced.

(6) On nutrient agar medium (cultured at 27°C): Growth is pale yellowish brown, and no aerial mycelia develop. No soluble pigment is produced.

(7) On yeast extract-malt extract agar medium (ISP-2, cultured at 27°C): Growth is colorless to pale yellowish brown (3 ie, camel), and white to pale brown (4 ec, Bisque - 5 ec, Dusty peach) aerial mycelia develop on the growth. Slightly brownish soluble pigment is produced.

(8) On oatmeal agar medium (ISP-3, cultured at 27°C): Growth is pale yellow to pale yellowish brown, and white to pale pink (4 ca, Flesh pink) aerial mycelia develop on the growth. Slightly brownish soluble pigment is produced.

(9) On glycerol-nitrate agar medium (cultured at

27°C): Growth is pale yellowish brown (2 gc, Bamboo), and white aerial mycelia develop on the growth. No soluble pigment is produced.

(10) On starch agar medium (cultured at 27°C): Growth is pale yellow, and pale pink (5 ca, Flesh pink - 4 ca, Flesh pink) aerial mycelia develop slightly on the growth. No soluble pigment is produced.

(11) On calcium maleate agar medium (cultured at 27°C): Growth is colorless, and white to brownish white aerial mycelia develop on the growth. No soluble pigment is produced.

(12) On cellulose agar medium (filter paper-containing synthetic liquid, cultured at 27°C): Growth is colorless, and no aerial mycelia develop. No soluble pigment is produced.

(13) On gelatin stab culture medium: On simple gelatin medium (cultured at 20°C), growth is pale brown, no aerial mycelia develop, and brown soluble pigment is produced. On glucose-peptone-gelatin medium (cultured at 27°C), growth is pale brown, no aerial mycelia develop, and dark brown soluble pigment is produced.

(14) On skimmed milk medium (cultured at 37°C): Growth is pale brown, no aerial mycelia develop, and brownish soluble pigment is produced.

3. Physiological properties

(1) Temperature range for growth: Tests for growth are carried out at temperatures of 20, 24, 27, 30, 37 and 50°C using a glucose-asparagine agar medium. Growth occurs at all of these temperatures, except 50°C, and the optimum temperature is considered to be about 24 to 30°C.

(2) Liquefaction of gelatin (15 % simple gelatin, cultured at 20°C; glucose-peptone-gelatin, cultured at 27°C): On each of simple gelatin medium and glucose-peptone-gelatin medium, liquefaction begins after about 10 days of cultivation. The liquefactive strength is

intermediate for both media.

(3) Hydrolysis of starch (tested on starch-inorganic salts agar medium, and starch agar medium, each cultured at 27°C): Hydrolysis begins after about 3 days of cultivation for starch-inorganic salts agar medium, and after about 5 days of cultivation for starch agar medium. The hydrolytic strength is intermediate to high.

(4) Coagulation and peptonization of skimmed milk (cultured at 37°C): Coagulation begins after about 10 days of cultivation, and finishes immediately, followed by the start of peptonization. Peptonization is completed after about 21 days of cultivation. The strength is intermediate to high for both of coagulation and peptonization.

(5) Production of melanoid pigment (tested on tryptone-yeast extract-broth medium, ISP-1; peptone-yeast extract-iron agar medium, ISP-6; tyrosine agar medium, ISP-7; each cultured at 27°C): Melanoid pigment production is examined using tryptone-yeast extract-broth medium and peptone-yeast extract-iron agar medium. Clear melanoid pigment production cannot be observed for tyrosine agar medium.

(6) Utilization of carbon sources (tested on Pridham-Gottlieb agar medium, ISP-9, cultured at 27°C): D-glucose, L-arabinose, and D-xylose are utilized for growth. D-fructose, sucrose, inositol, L-rhamnose, raffinose or D-mannitol is not utilized.

(7) Dissolution of calcium maleate (tested on calcium malate agar medium, cultured at 27°C): Calcium maleate around the growth is dissolved after about 7 days of cultivation. The dissolving strength is intermediate to high.

(8) Nitrate reduction (tested on 1.0 % potassium nitrate containing peptone water, ISP-8, cultured at 27°C): Reduction is positive.

The caracteristics described above can be summarized as follows:

The strain MF118-A5 belongs to the genus Streptomyces, and 2,6-diaminopimelic acid contained in its cell wall is of the LL-type. No sporangium is observed, aerial myceia are straight, and no whirls are seen. The surface of spores is smooth. Growths on various media are colorless to pale yellowish brown, and white to pale pink or pale brown aerial mycelia develop on the growths. There is little production of any soluble pigment. Melanoid pigment production is positive, the proteolytic strength is intermediate, and the starch-hydrolyzing activity is intermediate to high.

If these properties are compared with those of known species, the strain MF118-A5 is close to the following two microorganisms: Actinomyces roseoviridis [Reference (1): International Journal of Systematic Bacteriology, Vol. 18, page 372, 1968, Reference (2): Gause, G. F. et al, Zur Klassifizierung der Actinomyceten, page 43, VEB Gustav Fischer Verlag, Jena, 1958)] and Streptomyces lavendulae (International Journal of Systematic Bacteriology, Vol. 18, page 138, 1968).

The table below summarizes the results of comparative tests on the ISP strains (kept in the present applicant's research institute) corresponding to the above two species and the strain MF118-A5.

| | MD 118-A5 | Actinomyces roseoviridis IMCS-0149 (ISP 5175) | Streptomyces lavendulae IMCS-0169 (ISP 5069) |
|---|---|---|---|
| Shape of aerial myceria | Straight | Straight | Straight to hooky |
| Surface of spore | Smooth | Smooth | Smooth |
| Color of aerial myceria | White to pale pink, white to pale brown | White to brownish white, white to pale pink | Grayish white to bright brownish gray |
| Color of growth | Colorless to pale yellowish brown | Colorless to pale yellowish brown | Colorless to pale brown |
| Soluble pigment | − | − | − |
| Production of melanoid pigment | | | |
| ISP-1 | + | + | + |
| ISP-6 | + | + | + |
| ISP-7 | − | − | − |
| Hydrolysis of starch | + | + −* | + |
| Coagulation of milk | + | + −* | + |
| Peptonization of milk | + | + −* | + |
| Liquefaction of gelatin | | | |
| Simple gelatin | + | + | + |
| Glucose-peptone-gelatin | + | + | + |
| Reduction of nitrate | + | + | + |
| Utilization of carbon sucrose | | | |
| D-glucose | + | + | + |
| L-arabinose | + | + | − |
| D-xylose | + | + | − |
| D-fructose | − | − | − |
| Sucrose | − | − | − |
| Inositol | − | − | − |
| L-rhamnose | − | − | − |
| Raffinose | − | − | ± ∓** |
| D-mannitol | − | − | − |

±: Probably utilized.
∓: Not utilized, or slightly utilized for growth.
*: Disclosed in Reference (2).
**: Disclosed in the reference.

As shown in the above table, MF118-A5 and Streptomyces lavendulae IMCS-0169 (ISP 5069) are different in the shape and color of aerial mycelia, and the utitilization of carbon sources. Actinomyces roseoviridis IMCS-0149 (ISP 5175), on the other hand, is described in Reference (2) as negative for the hydrolysis of starch and for the coagulation and peptonization of milk, but has been found in the comparative experiments to be positive for these reactions, as in the case of MF118-A5. Thus, Actinomyces roseoviridis IMCS-0149 and MF118-A5 are in good agreement.

Accordingly, MF118-A5 was identified as *Streptomyces roseoviridis* MF118-A5.

The process of this invention will be described in greater detail below.

Nutritional sources for use in cultivating the histargin-producing strain according to the process of this invention may be any known nutritional sources that can be utilized by ordinary microorganisms. For example, commercially available fats and oils and carbohydrates, such as glycerol, glucose, lactose, sucrose, starch, maltose and molasses, can be used as carbon sources. Examples of nitrogen sources are peptone, meat extract, corn steep liquor, cotton seed meal, peanut meal, soybean flour, corn gluten meal, fish meal, yeast extract, N-Z amine, casein, sodium nitrate, amonium nitrate, and ammonium sulfate that are on the market.

Examples of inorganic nutritional sources are sodium chloride, phosphates, calcium carbonate and magnesium sulfate. Particularly preferred components of media are glycerol, dextrin, etc. as carbon sources, and soybean peptone, yeast extract, ammonium sulfate, etc. as nitrogen sources. It is preferred to use media containing 2.0 % of glycerol, 2.0 % of dextrin, 1.0 % of soybean peptone, 0.3 % of yeast extract, 0.2 % of ammonium sulfate, 0.2 %

of calcium carbonate, and 0.01 % of the anti-foaming agent KM70 (trade mark for a product of Shin-etsu Chemical Co., Ltd., Japan).

Liquid culture is a preferred way of mass-producing histargin. The cultivation temperature is that at which the histargin-producing strain grows and produces histargin. A suitable temperature is 20 to 40°C, preferably 25 to 35°C. Cultivation is usually continued until histargin is produced and accumulated in sufficient amounts in the culture broth. An example of the cultivation will be given below.

120 ml of a medium (adjusted to a pH of 7.4) containing a 2.0 % of glycerol, 2.0 % of dextrin, 1.0 % of soybean peptone, 0.3 % of yeast extract, 0.2 % of ammonium sulfate, 0.2 % of calcium carbonate and 0.01 % of the anti-foaming agent KM70 was put in a 500 ml Sakaguchi flask, and sterilized. A loopful of spores from a slant culture of the histargin-producing strain was inoculated into the medium, and shake-cultured aerobically at 27°C. Accumulation of histargin was observed after 45 to 150 hours of cultivating. Histargin was produced in as good amounts by fermentor culture as by shake culture. For example, 120 liters of a medium was put in a 200 liters fermentor, and sterilized. Five liters of a cultured broth containing the histargin-producing strain was inoculated into the medium. The inoculated medium was incubated with stirring at 250 rpm under aeration with 90 liters/minute of sterile air. Under these conditions, production of histargin reached its maximum after 68 hours of cultivation.

Histargin in the cultivation and purification steps was traced by measuring anti-carboxypeptidase B activity. Anti-carboxypeptidase B activity was measured in the following way according to an improved method based on the angiotensin I transferase activity determination

method described in M.Hayakari et al., "Analytical Biochemistry," 84, 361-369, 1978.

A mixed solution was prepared by adding 0.25 ml of 0.05M Tris-hydrochloric acid buffer solution (pH 8.0) and 0.15 ml of a solution containing a histargin sample to 0.05 ml of 0.05M hippuryl-L-lysine (a product of Peptide Institute). The mixed solution was heated for 3 minutes at 37°C, and 0.05 ml of an aqueous solution of 1 µg/ml of purified carboxypeptidase B (a product of Boehringer-Mannheim obtained from the pancreas of pigs) was added to the mixed solution. To the resulting mixture was added 0.03 ml of 1N sodium hydroxide to terminate the reaction. Fifteen minutes later, 2 ml of a 1 % methyl cellosolve solution of cyanuric chloride was added for a color reaction. After the mixture was allowed to stand for 15 minutes at room temperature, the absorbance (a) at 382 nm was measured. Separately, there was measured the blank absorbance (b) of the same reaction mixture except that the histargin sample was not contained. The percent inhibition against carboxypeptidase B was calculated from the equation $[(b - a)/b] \times 100$. According to this method, pure histargin monohydrate in a concentration of 12 µg/ml gave a 50 % inhibition ($IC_{50}$) against carboxypeptidase B.

As shown in the Examples to be given later, histargin was also found to exhibit inhibitory activity against enkephalinase B that exists in the brain of animals and participates in the physiological mechanism of pain.

Histargin is present in the filtrate of the culture broth of the histargin-producing strain, as well as in the bacterial cells contained in the culture broth. To recover histargin from the culture broth in good yields, it is suitable to employ a method comprising adsorbing the culture broth filtrate onto an adsorbent, and releasing histargin from the adsorbent. Examples of the adsorbent are activated carbon, ion-exchange resins, and

nonionic adsorbent resins.

For instance, histargin is adsorbed to Amberlite IRC50 ($H^+$ type), and eluted with 0.5N hydrochloric acid containing 50 % acetone. In detail, a column is packed with Amberlite IRC50 ($H^+$ type) whose volume is 8 % of the volume of the culture broth filtrate. Then, the culture broth filtrate is adsorbed onto the column. The column is washed with water, and then with a 50 % aqueous solution of acetone.

Elution of the column with 0.5N hydrochloric acid containing 50 % acetone gives active fractions. The combined active fraction is neutralized with soidum hydroxide, and concentrated under reduced pressure to obtain a crude concentrate of histargin. Then, the concentrate is diluted with water, and 0.18 % by weight, based on the weight of the culture broth filtrate, of activated carbon is added to the dilution. The mixture is stirred to adsorb histargin onto to activated carbon. After separation by filtration, the activated carbon is washed with water, and eluted with a 90 % aqueous solution of methanol (adjusted to pH 2 with hydrochloric acid). The eluate is concentrated under reduced pressure to obtain a crude powder of histargin. Chromatography on an ion exchange resin may be used to purify the crude histargin. Chromatography on Dowex 50W is particularly useful for the purification. According to this technique, the crude histargin can be purified using pyridine-acetic acid buffer solution.

Histargin obtained in the above manner by this invention was examined for biological activities or pharmacological actions. This substance was found to have an analgesic action, and the action to potentiate cell-mediated immunity. The following tests describe the biological activities of histargin.

Test A

This test shows the effect of histargin on cell-mediated immunity in normal mice. This effect was investigated using as its index the degree of delayed-type hypersensitivity (D.T.H.) to sheep red blood cell (SRBC) injectd intravenously as an antigen (see J. Exp. Med. 139, 1529-1539, 1974).

A suspension of $10^5$ SRBCs in 0.05 ml of physiological saline solution was injected intravenously into Slc-$CDF_1$ mice (female, 9 weeks old). Simultaneously, histargin monohydrate in doses of 5, 0.5, 0,05 and 0.005 mg/kg was administerd once intraperitoneally as a solution dissolved in physiological saline solution.

Four days after administration, a suspension of $10^8$ SRBCs in 0.05 ml of physiological saline solution was injected subcutaneously to the footpads to induce D.T.H. Twenty-four hours after the subcutaneous injection, footpad swellings (increases in the foot thicknesses) were measured with a caliper. The mean footpad swelling in control animals receiving SRBCs and physiological saline solution but without the test compound was designated as C, which was set at 100 %. The mean footpad swelling in animals treated with SRBCs, physiological saline solution, and the test compound was designated as T. The ratio of T to C was calculated to evaluate the effect of the test compound to potentiate cell-mediated immunity. The results are shown in Table 1.

Table 1

| Test compound | Dose (μg/kg) | T/C (%) | Mean footpad swelling + S.E. (x 0.1 mm) |
|---|---|---|---|
| Histargin monohydrate | 5 | 96 | 6.9 ± 0.5 |
| | 50 | 110 | 7.9 ± 0.6 |
| | 500 | 118 | 8.5 ± 0.8 |
| | 5000 | 107 | 7.7 + 0.8 |
| Control (physiological saline solution) | – | 100 | 7.2 + 0.5 |
| Bestatin (for comparison) | 5 | 119 | 8.6 ± 0.7 |
| | 50 | 136 | 9.8 ± 0.7 |
| | 500 | 125 | 9.0 + 0.9 |

The above table shows histargin to be a substance which potentiates cell-mediated immunity in normal animals. Accordingly, histargin obtained by this invention is useful as an immunopotentiator. It is also useful as an antineoplastic immunomodulator and finds use as an adjuvant to various chemotherapeutic agents against cancer.

Test B

This test shows the action of histargin to potentiate analgesia produced by morphine.

Wistar rats were intraperitoneally administered 0.5 mg/kg of morphine, and classified into rats responsive to an analgesic action of morphine and rats unresponsive to this action. This classification test complied with the Showa Igaku Zasshi (Showa Medical Journal), Vol. 39, pp. 573-542, 1979. The morphine-unresponsive rats were used in studies of whether histargin will potentiate morphine-induced analgesia.

More than about 1 week after the above classification test, a solution of histargin monohydrate (100

mg/kg) of this invention dissolved in physiological saline solution was administered intraperitoneally. Then, 0.5 mg/kg of morphine was administered. The analgesic effect of the test compound was evaluated by the tail-flic technique wherein a pain threshold was measured as follows:

That portion of the tail which was about 1 cm apart from its tip was painted black, and radiant heat was applied there. The latent time to the escape reflex of the tail was measured. For the control group, the amount of geat was adjusted so that the latent time to the escape reflex of the tail was about 2.0 seconds on the average. Latent times longer than 7.0 seconds were not measured in order to avoid any damages to the skin of the tail. Measurement was performed at intervals of 15 minutes, with 5 determinations taken at each time of measurement. The average value of 5 determinations was taken as the latent time to the escape reflex of the tail in each animal.

The latent times to the tail in the control group (0.5 mg/kg morphine only) were compared with those for the treatment group (100 mg/kg histargin sample + 0.5 mg/kg morphine) to evaluate the analgesic effect in the two groups. The results are shown in Table 2.

Table 2

| Test compound | Dose of test compound (mg/kg) | Potentiation of morphine-induced analgesia (%) |
|---|---|---|
| histargin monohydrate | 100 | 33.8 |
| Bestatin (for comparison) | 250 | 19.9 |
| Morphine only | 0.5 | 0 |
| Ditto | 2 | 28.4 |
| Ditto | 3 | 35.1 |

The above results demonstrate histargin to be a substance potentiating morphine-induced analgesia in normal animals.

Test C

This test shows the activity of histargin to inhibit enkephalinase B.

Enkephalinase B was obtained by homogenizing the brains of crab-eating monkeys, fractionating the homogenate, and purifying soluble fractions in accordance with the method of Hazato et al. (Biochemical and Biophysical Research Communications, Vol. 105, No. 2, pp. 470-475, 1982). The inhibitory activity of histargin against enkephalinase B was measured by the method of Hasato et al. described in the same reference.

Ten µmol of methionine enkephalin (Tyr-Gly-Gly-Phe-Met) having the terminal tyrosine partially tritiated was used as a substrate. This substrate was hydrolyzed with enkephalinase B in 100 µl of 25 mN Tris-hydrochloric acid buffer solution (pH 7.0). The segment, Tyr-Gly, resulting from hydrolysis was separated using a column of Porapak Q (a product of Whatman), and determined quantitatively based on its radioactivity. The results obtained when the above reaction was carried out in the presence of histargin were compared with those obtained when the reaction was performed in the absence of histargin. Based on the comparison, the enkephalinase B-inhibiting activity of histragin was evaluated. Histargin monohydrate in a concentration of 100 µg/ml was found to give a 92 % inhibition against enkephalinase B.

Thus, histargin can be highly expected to show an analgesic action even when used alone.

Acute toxicity studies in mice showed 1 g/kg of histargin monohydrate to cause no deaths when administered intravenously. Histargin is thus considered to be a substance having low toxicity.

Analgesics or analgesia-potentiators containing histargin as the active ingredient can be prepared by combining histargin or its pharmaceutically acceptable salts or esters or hydrates with carriers that are widely used. Histargin or its pharmaceutically acceptable salts or esters or hydrates may also be blended with various chemotherapeutic agents. Examples or the salts of histargin are salts formed between the carboxyl groups of histargin and pharmaceutically acceptable cations, such as the cations of alkali metals (e.g. sodium, potassium) and alkaline earth metals (e.g. calcium, magnesium). The salts of histargin also include acid-addition salts formed between one or both of the imino and guanidino groups of histargin and pharmaceutically acceptable inorganic acids (e.g. hydrochloric acid) or organic acids (e.g. acetic acid). Examples of the esters of histargin include alkyl esters taht can be easily metabolized and decomposed in vivo.

The compound and pharmaceutical compositions of this invention may be administered as oral agents, injections or rectal suppositories. To prepare injections, pH adjustors, buffers, stabilizers, and excipients are aded to said compounds as active ingredients. The mixtures are then freeze-dried by customary methods to make lyophilized injections. Subcutaneous, intramuscular and intravenous injections can be prepared by adding pH adjustors, buffers, stabilizers, isotonizers, and local anesthetics to the compounds as active ingredients, and treating the mixtures by customary methods. To make solid preparations for oral administration, excipients, and if desired, binders, disintegrators, lubricants, colorants, taste correctives and odor correctives are added to the compounds as active ingredients. Then, the mixtures are

formed into tablets, coated tablets, granules, powders, and capsules by customary methods. To prepare liquid preparations for oral administration, taste correctives, buffers, stabilizers, and odor correctives are added to the compounds as active ingredients, followed by forming the mixtures into syrups and dry syrups by customary methods.

To produce rectal suppositories, excipients, and if desired, surfactants are added to the compounds as active ingredients, and then, the mixtures are made into suppositories by customary methods.

The doses of histargin vary according to symptoms and objectives of treatment. In adults, histargin 0.02 to 2000 mg t.i.d. can be given.

The production of histargin according to this invention is described in detail with reference to the following Examples, but this invention is in no way limited to these examples. This is because it is easy to modify the methods disclosed in the present specification in the light of the disclosures concerning the physical and chemical properties of histargin, as well as the methods of producing and purifying it.

Example 1

A medium was prepared according to the following recipe:

| | |
|---|---|
| Glycerol | 2.0 % |
| Dextrin | 2.0 % |
| Soybean peptone | 1.0 % |
| Yeast extract | 0.3 % |
| Ammonium sulfate | 0.2 % |
| Calcium carbonate | 0.2 % |
| Antifoaming agent KM70 | 0.01 % |
| pH: | 7.4 |

A loopful of the histargin-producing strain *Streptomyces roseoviridis* MF118-A5 (FERM P-7043) from its slant

culture was used to inoculate 500 ml Sakaguchi flasks each containing 120 ml of the above medium which had been sterilized for 20 minutes at 120°C. The inoculated medium was aerobically shake-cultured with 130 reciprocations/ minute at 27°C.

The amount of histargin produced was examined with the passage of time by measuring the anti-carboxypeptidase B activity of the cultured broth. The concentration of histargin in the cultured broth that was determined by the carboxypeptidase B-inhibiting activity reached its peak after 4 days of cultivation, and maintained constant high values until after 6 days of cultivation. Thereafter, its values gradually decreased.

Example 2

5 ml of the cultured broth as primary inoculum that had been prepared by 28 hours of cultivation under the same conditions as shown in Example 1 was inoculated into the same medium as described in Example 1. The inoculated medium was fermented for 96 hours under the same conditions as in Example 1 to obtain 60 liters of a fermented mash. The fermented mash was filtered using diatomaceous earth as a filter aid to obtain 56 liters of the filtrate. The carboxypeptidase B-inhibiting activity ($IC_{50}$) of the filtrate was 90 µl/ml.

56 liters of the filtrate was adsorbed onto a 4.5 liter column of the ion exchange resin Amberlite 50 ($H^+$ type). The column was washed with 15 liters of water, and then with 15 liters of a 50 % aqueous solution of acetone. Then, the column was eluted with 0.5N hydrochloric acid containing 50 % acetone, thereby to obtain 250 ml each of fractions. Active fractions were collected, whereafter the combined active fraction was neutralized with 2N sodiumhydroxide, and concentrated under reduced pressure to 400 ml. Water was added to the concentrate to make a 4 liter mixture, followed by

putting therein 90 g of activated carbon. The mixture was stirred for 60 minutes to adsorb histargin onto the activated carbon. The activated carbon was separated by filtration, and washed with 5 liters of water. Then, the activated carbon was put in 2 liters of a 90 % aqueous solution of methanol (adjusted to pH 2 with hydrochloric acid), and the solution was stirred for 30 minutes to elute histargin from the activated carbon. The solution was filtered, and the filtrate was concentrated to dryness under reduced pressure to obtain 5.3 g of a crude powder. The carboxypeptidase B-inhibiting activity ($IC_{50}$) of this crude powder was 55.6 µg/ml.

The crude powder was dissolved in 500 ml of 0.2M pyridine-acetic acid buffer solution with a pH of 3.2. The solution was adsorbed onto a 300 ml column of the ion exchange resin Dowex 50W (pyridinium type, x 4, 100-200 mesh) equilibrated with the same buffer solution as described above. The column was washed with 0.5M pyridine-acetic acid buffer solution with a pH of 4.6, and eluted with 1M pyridine-acetic acid buffer solution with a pH of 5.0. Active fractions were collected, and the combined active fraction was concentrated to dryness under reduced pressure to obtain 1.5 g of a crude powder. The carboxypeptidase B-inhibiting activity ($IC_{50}$) of the crude powder was 35.3 µg/ml.

The crude powder was dissolved in 10 ml of a 10 % aqueous solution of ammonium acetate. The solution was adsorbed onto the high-performance liquid chromatographic reversed-phase column Partisil-10-ODS-3/Magnum 20 (product of Whatman) that had been equilibrated with a 10 % aqueous solution of ammonium acetate. The column was eluted with a 10 % aqueous solution of ammonium acetate at a rate of 8 ml/minute. The eluate was obtained as fractions with a volume of 8 ml each. Active fractions (Fraction

Nos. 40 to 53) were combined and diluted with water to 500 ml. The dilution was adsorbed onto a 300 ml column of the ion exchange resin Dowex 50W ($H^+$ type, x 4, 100-200 mesh). The column was washed with 1 liter of water, and eluted with 1.5N ammonia water. Active fractions were collected, and the combined active fraction was concentrated to dryness under reduced pressure to obtain 406 mg of pure stargin monohydrate which had a carboxypeptidase B-inhibiting activity ($IC_{50}$) of 12 µg/ml.

The attached drawing shows the infrared absorption spectrum of histargin monohydrate according to this invention.

Claims

1. A compound of the formula

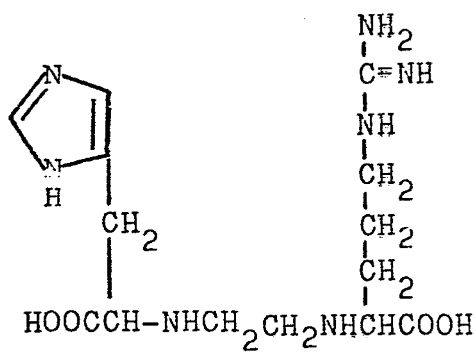

or a physiologically acceptable salt or ester or the hydrate thereof.

2. A process for the preparation of a compound of the formula

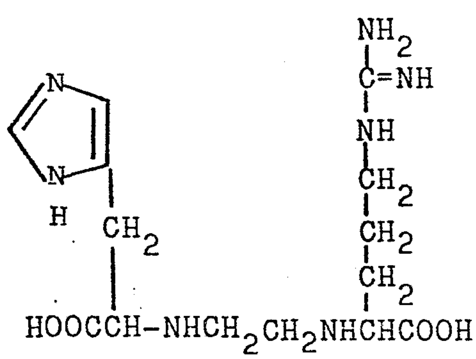

which comprises cultivating a strain of the genus Streptomyces in a suitable culture medium to produce and accumulate this compound in the culture broth and then recovering it therefrom by conventional chromatographic methods.

3. The process as defined in claim 2 wherein the strain Streptomyces roseoviridis MF118-A5 (FERM-P No. 7043) is used.

4. The process as defined in claims 2 and 3 wherein the cultivation is carried through in a liquid culture medium comprising nutritional carbon sources, nitrogen sources and optionally inorganic salts and/or anti-foaming agents at a temperature of from 20 to 40°C and a cultivation time from 45 to 150 hours, and the produced histargin is recovered from the culture broth by chromatography with activated carbon, ion-exchange resins or non-ionic adsorbent resins.

5. A pharmaceutical composition containing as its active ingredient the compound histargine of the formula as defined in claim 1 or a physiologically acceptable salt or ester, or the hydrate thereof in association with a pharmaceutically acceptable carrier.

6. The compound histargin of the formula as defined in claim 1 for use as a medicament.

7. The strain Streptomyces roseovirides FERM-P No. 7043.

Claims for Austria:

1. A process for the preparation of a compound of the formula

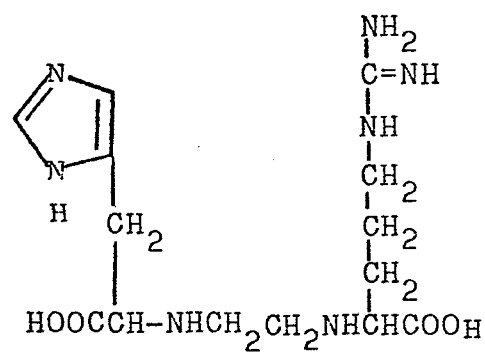

which comprises cultivating a strain of the genus Streptomyces in a suitable culture medium to produce and accumulate this compound in the culture broth and then recovering it therefrom by conventional chromatographic methods.

2. The process as defined in claim 2 wherein the strain Streptomyces roseoviridis MF118-A5 (FERM-P No. 7043) is used.

3. The process as defined in claims 2 and 3 wherein the cultivation is carried through in a liquid culture medium comprising nutritional carbon sources, nitrogen sources and optionally inorganic salts and/or antifoaming agents at a temperature of from 20 to 40°C, a cultivation time from 45 to 150 hours, and the produced histargin is recovered from the culture broth by chromatography with activated carbon, ion-exchange resins or non-ionic adsorbent resins.

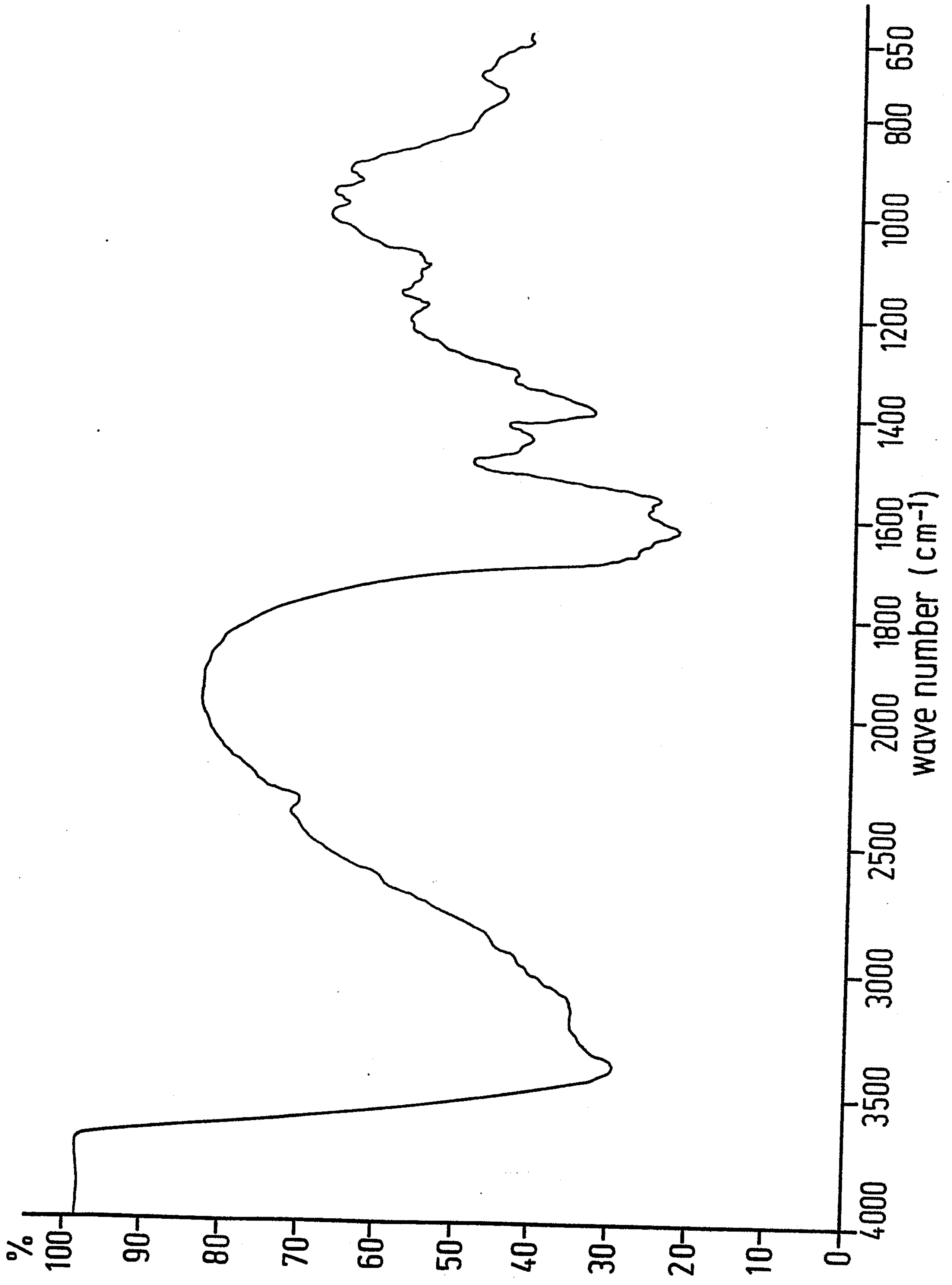
%
100
90
80
70
60
50
40
30
20
10
0
4000
3500
3000
2500
2000
1800
1600
1400
1200
1000
800
650
wave number (cm⁻¹)